# EUROPEAN PATENT SPECIFICATION

(11) **EP 2 846 770 B1**
(45) Date of publication and mention of the grant of the patent: **13.06.2018**
(21) Application number: 13730352.5
(22) Date of filing: 10.05.2013
(51) Int. Cl.: A61K 9/08, A61K 31/436, A61P 25/28

(54) **INTRATHECAL ADMINISTRATION OF MTOR INHIBITORS FOR THE THERAPY OF NEURODEGENERATIVE, NEUROINFLAMMATORY AND NEUROONCOLOGIC DISEASES**
INTRATHEKALE ADMINISTRATION VON MTOR INHIBITOREN ZUR BEHANDLUNG NEURODEGENERATIVER, NEUROINFLAMMATORISCHER UND NEUROONKOLOGISCHER KRANKHEITEN
ADMINISTRATION INTRATHÉCALE DES INHIBITEURS DE MTOR POUR LE TRAITEMENT DES MALADIES NEURO-DEGENERATIVES, NEURO-INFLAMMATOIRES ET NEURO-ONCOLOGIQUES

(30) Priority: 11.05.2012 IT MI20120814
(43) Date of publication of application: 18.03.2015
(73) Proprietor: Dolcetta, Diego, 36100 Vicenza (IT)
(72) Inventor: CASSANO, Tommaso, I-00161 Roma (IT); GIOVAGNOLI, Stefano, I-06124 Perugia (IT); MAGINI, Alessandro, I-06122 Perugia (IT); EMILIANI, Carla, I-06070 Cenerente (Perugia) (IT)
(74) Representative: Biggi, Cristina
(86) International application number: PCT/IB2013/053792
(87) International publication number: WO 2013/168131

(56) References cited:
- US-A1- 2009 274 764
- CREWS LESLIE ET AL: "Selective Molecular Alterations in the Autophagy Pathway in Patients with Lewy Body Disease and in Models of alpha-Synucleinopathy", PLOS ONE, vol. 5, no. 2, E9313, February 2010 (2010-02), pages 1-16, XP002685164, ISSN: 1932-6203
- BOVE JORDI ET AL: "Fighting neurodegeneration with rapamycin: mechanistic insights", NATURE REVIEWS NEUROSCIENCE, vol. 12, no. 8, August 2011 (2011-08), pages 437-452, XP002685223,
- DELLO RUSSO C ET AL: "Involvement of mTOR kinase in cytokine-dependent microglial activation and cell proliferation", BIOCHEMICAL PHARMACOLOGY, vol. 78, no. 9, 1 November 2009 (2009-11-01), pages 1242-1251, XP026613861, PERGAMON, OXFORD, GB ISSN: 0006-2952, DOI: 10.1016/J.BCP.2009.06.097 [retrieved on 2009-07-01]
- WINSLOW A R ET AL: "Autophagy in neurodegeneration and development", BIOCHIMICA ET BIOPHYSICA ACTA. MOLECULAR BASIS OF DISEASE, AMSTERDAM, NL, vol. 1782, no. 12, 1 December 2008 (2008-12-01), pages 723-729, XP025691382, ISSN: 0925-4439, DOI: 10.1016/J.BBADIS.2008.06.010 [retrieved on 2008-07-01]
- SANDSMARK D K ET AL: "Mammalian target of rapamycin: master regulator of cell growth in the nervous system.", HISTOLOGY AND HISTOPATHOLOGY, vol. 22, no. 8, August 2007 (2007-08), pages 895-903, XP002685224, ISSN: 1699-5848
- MICHAL YALON ET AL: "Regression of subependymal giant cell astrocytomas with RAD001 (Everolimus) in tuberous sclerosis complex", CHILD'S NERVOUS SYSTEM, SPRINGER, BERLIN, DE, vol. 27, no. 1, 12 August 2010 (2010-08-12), pages 179-181, XP019869880, ISSN: 1433-0350, DOI: 10.1007/S00381-010-1222-Y
- MARTA PEREK-POLNIK ET AL: "Effective everolimus treatment of inoperable, life-threatening subependymal giant cell astrocytoma and intractable epilepsy in a patient with tuberous sclerosis complex", EUROPEAN JOURNAL OF PAEDIATRIC NEUROLOGY, SAUNDERS, PHILADELPHIA, US, vol. 16, no. 1, 18 September 2011 (2011-09-18), pages 83-85, XP028346213, ISSN: 1090-3798, DOI: 10.1016/J.EJPN.2011.09.006 [retrieved on 2011-09-23]

## Description

### FIELD OF THE INVENTION

The present invention relates to the use of inhibitors of the enzyme mTOR kinase (mammalian target of rapamycin) in the treatment of tuberous sclerosis, Alzheimer's disease and primary progressive aphasia by intrathecal administration, preferably intraventricular, of these inhibitors. The mTOR inhibitor used in the present invention is everolimus.

### BACKGROUND OF THE INVENTION

The founder of the family of mTOR inhibitors, rapamycin or sirolimus, was initially proposed as an antibiotic and antifungal drug of the macrolide family. It was later discovered to be in possession of powerful immunosuppressive properties. These became the principal application of the drug. mTOR has a key role in adapting cell functions to environmental conditions. In the presence of energy substrates, nutrients and amino acids, it is activated and promotes, for example, protein synthesis and mitosis, whereas it is inhibited during fasting, i.e. when these are lacking. What is promoted in this case is autophagy, i.e. the catabolic process in which the substrate consists of elements belonging to the cell itself (source of amino acids and energy). mTOR inhibitors thus promote energy savings. mTOR is the catalytic subunit of 2 molecular complexes: mTORC1 and mTORC2. mTORC1 integrates the 4 signals deriving from nutrients, growth factors, energy and stress, and of the 2 it is more sensitive to rapamycin. mTORC2 is sensitive to indirectly to rapamycin. This cascade, evolutionarily highly conserved, has the purpose of maintaining the energy balance and adapting cell functions to it.

The effectiveness of mTOR inhibitors (mammalian target of rapamycin) has been amply demonstrated in the treatment of numerous pathologies involving the central nervous system (CNS).
This is true for many neurodegenerative, neuro-oncologic and neuroinflammatory diseases. A number of clinical studies have been or are being carried out to evaluate the benefits of mTOR inhibitors in the treatment of tuberous sclerosis (TS), a genetic tumoural syndrome. Since 2006 (Franz DN et al., Ann. Neurol.) we have known that mTOR inhibitors are effective in inducing the regression of the astrocytomas typical of this condition (Subependymal Giant Cell Astrocytomas, SEGAs). In 2008, Ehninger et al. (Nat Med 2008) found a reversal of the learning deficit in a TS mouse model following administration of mTOR inhibitors, notwithstanding that the treatment was started after development had stopped. TS is associated with profound alterations in cerebral cytoarchitecture, and this is considered the cause of epileptic manifestations (often not treatable) and severe cognitive and behavioural disorders, frequent in children with TS, who are commonly classified within the autism spectrum.
All this led Ehninger and Silva (Trends in Mol. Med., 2011) to hypothesize a benefit of treatment with an mTOR inhibitor in autism spectrum disorders.
SEGAs, together with renal neoplasias (angiomyolipoma, cysts and renal cell carcinoma) are the most frequent cause of death in TS (Shepherd et al, Mayo Clin. Proc., 1991). Therefore, the administration of mTOR inhibitors in patients with TS has a beneficial effect not only in inducing the regression of SEGAs - by exploiting the anti-mitotic effect - but also - through different mechanisms - on patients' learning, memory, convulsions and behaviour.

mTOR inhibitors have also been used in animal models of Alzheimer's disease (AD).
In January 2012, S. Oddo summarized (Front. Biosci. (Schol Ed.)) the results of recent years on the effects of administering an mTOR inhibitor (rapamycin, also known as sirolimus) to mouse models of Alzheimer's disease.
In 2010 his group (Caccamo et al, Biol Chem) published the effect of orally administering mTOR inhibitors to a mouse model of Alzheimer's disease (3xTg-AD mouse); they observed an improvement in the accumulation of both Tau neurofibrils and amyloid-beta in the brain (together with neuronal loss, key anatomopathological characteristics of Alzheimer's), with an improvement in learning and memory. The effect seems to be due to the inducement of autophagy mediated by the mTOR inhibitor. The push toward autophagy induces intracellular degradation of the pathological accumulations of protein in Alzheimer's. The link between this and the beneficial cognitive effect observed in Alzheimer's is not yet completely understood.
In another article published by his group (Majumder et al., PLoS One 2011), the mTOR inhibitor is proposed as an effective therapeutic treatment if administered at the start of the disease's progression.
mTOR inhibitors could also be used in the treatment of neuroinflammatory diseases. Roberto Furlan's group (Esposito M. et al, J neuroimmunology 2010) has demonstrated the effectiveness of treatment with mTOR inhibitors also in animal models of neuroinflammatory diseases such as experimental allergic encephalitis (EAE), the animal model of multiple sclerosis (MS).
One of the treated models, created by immunizing SJL mice with a peptide (amino acids 139-151), part of the myelin proteolipid protein (PLP), represents the human disease better than others, as it expresses a clinical form characterized, as in patients, by attacks and remissions occurring at close intervals, with a gradual worsening of the disability. The administration of rapamycin before the disease manifested itself was extremely effective, as it substantially inhibited its onset and successive relapses. Administration after the first attack reduced the severity and number of subsequent attacks. The trial also demonstrated that when the drug was suspended, the disease resumed its course, as in untreated subjects.

One form of dementia in which the inflammatory aspect seems important is primary progressive aphasia (PPA).
Initially described by M. Mesulam at the end of the 1980s, this form of frontotemporal dementia has long been associated with an autoimmune pathogenetic mechanism. It has been observed with a high incidence in patients who underwent vasectomy; anti-sperm antibodies are often found in male patients. They are also often found in patients of the female sex, for unknown reasons.
Decker and Heilman (Arch Neurol 2008) have reported significant benefits that were obtained through the administration of steroids in one case of PPA.
In a review of the results of autopsies performed on 60 cases of frontotemporal dementia, published by Kertesz et al. (Brain, 2005), the majority of PPA post-mortems showed the pathological characteristics of Alzheimer's (AD), suggesting that an AD-like degeneration very often represents the natural evolution of PPA.
Therefore, as regards both the initial inflammatory pathogenesis (mTOR inhibitors are above all powerful immunosuppressants), and the final AD-like evolution, the administration of a powerful immunosuppressive drug like an mTOR inhibitor seems to represent a highly interesting option.
There obviously exist no animal models of progressive aphasia.

The clinical conditions described above are only examples of diseases in which the effectiveness of administering an mTOR inhibitor has been clinically (TS) or pre-clinically demonstrated (AD and MS), or is simply likely (PPA). These applications can apparently be far from the main field mTOR inhibitors are used in: transplant immunology. The application on multiple sclerosis remains on this plane, but the application on tuberous sclerosis exploits the antimitotic effect, while that on Alzheimer's exploits the increase in autophagy.
The new applications, for example in the case of tuberous sclerosis, have given rise to large investments and clinical trials. In all the studies carried out, systemic administration led to an uncertain balance in the risk-benefit ratio, above all due to the main immunosuppressive action of the drug and the consequent undesirable side effects of an infectious type. In the N Eng J Med in 2010, Krueger DA reported the results of a phase II clinical study assessing the effect of the systemic administration of everolimus (mTOR inhibitor) on TS associated with astrocytomas (SEGAs). The studio, carried out on 28 patients affected by TS, reached the endpoint (volumetric reduction in SEGAs), but there were many adverse reactions, with a SEGAs), but there were many adverse reactions, with a severity grade of 1-3, most of them of infectious origin.
The problem of the side effects linked to the systemic administration of mTOR inhibitors is greatly felt in the art, and above all by patients who will have to take systemic drugs and tolerate the associated secondary effects for long periods, if not for their whole lives. It is for this reason that there are many publications aimed at supporting the tolerability of long-term treatment (e.g., Krueger DA, Neurology 2013) .
Yalon et al. Childs Nerv. Syst. (2011) 27:179-181 describes the oral administration of everolimus in the treatment of tuberous sclerosis complex.

### SUMMARY OF THE INVENTION

The present invention enables this problem to be overcome by providing a method for administering mTOR inhibitors, in particular everolimus, which serves to reduce the side effects that normally occur with systemic administration.

### BRIEF DESCRIPTION OF THE FIGURES

The invention will be described below in detail, also with reference to the appended figures, in which:
- Figure 1 shows: a) the levels of p70S6K (Total), phospho-p70S6K (pThr389) and actin in the brain, liver and spleen of control mice (CTRL) and mice treated with Everolimus (Ever), determined by immunoblotting analysis; the figure shows a western blot representing three different experiments; b) densitometric analysis of p70S6K, total and pThr389, normalized against actin (P < 0.01 (mice treated with Ever vs. CTRL mice), using a two-tailed Student t test for independent samples);
- Figure 2 shows the enzymatic activity of mTOR in the treated with Everolimus (Ever). (P < 0.01 (mice treated with Ever vs. CTRL mice), using a two-tailed Student t test for independent samples);

- Figure 3 shows the body weight of treated EAE mice and control mice;
- Figure 4 shows the clinical course of EAE mice treated with everolimus vs. the control mice;
- Figure 5 shows the mnestic performance of 6-month-old 3xTg-AD mice and control mice of the same sex and age; the control mice learn to find food in the space of about 8 days, whereas the AD mice have difficulty in learning the appropriate path within the time limit of 15 days.

### DETAILED DESCRIPTION OF THE INVENTION

The invention relates, therefore, to the use of mTOR inhibitors, in particular everolimus, for the treatment of neurodegenerative diseases, neuroinflammatory diseases and neuro-oncologic diseases, wherein said inhibitors are administered intrathecally. Preferably, the intrathecal administration takes place within the lateral ventricles (intraventricular administration).

The intrathecal administration according to the invention can be pulsed, preferably at regular intervals, or else continuous.

The intrathecal administration preferably takes place by means of a mechanical or electro-mechanical device, more preferably by means of an electro-mechanical infusion pump. The pump can consist, for example, of plastic devices (so-called "ports"), or mechanical ones (infusion pumps). Connected to the pump there is a catheter, which is inserted in the intrathecal space, or reaches the ventricular space. The pathologies that can be treated by intrathecal administration of mTOR inhibitors are Alzheimer's disease, tuberous sclerosis, multiple sclerosis and primary progressive aphasia. In particular, in the case of tuberous sclerosis with mTOR inhibitors, presently administered systemically, its neurological manifestations are treated with the proposed system.

Therefore, according to the invention, the mTOR inhibitor everolimus is used for treating neurodegenerative diseases, neuroinflammatory diseases and neuro-oncologic diseases by intrathecal administration, preferably intraventricular.

The mTOR inhibitors preferably usable in the intrathecal administration - preferably within the lateral ventricles - for the treatment of the aforesaid pathologies are selected from among: Everolimus, rapamycin (sirolimus), PF-04691502, ridaforolimus and temsirolimus.

Intrathecal administration of drugs is a technique that is already widely applied in various pathological conditions and has the advantage of limiting the systemic side effects. Intrathecal (IT) administration of analgesic or antispastic drugs is a well-established clinical practice that has been carried out since the 1980s, when infusion devices reached moderate costs and reduced dimensions and became sufficiently reliable. To date, only a few molecules have been approved by the FDA for this method of administration. Among them, we shall mention:
- Baclofen (antispastic, hydrophilic molecule)
- Morphine (analgesic, hydrophilic molecule)
- Bupivacaine (analgesic and anaesthetic, lipophilic molecule)
- Clonidine (antihypertensive and analgesic, lipophilic molecule)
- Ziconotide (analgesic, approved only for IT, hydrophilic molecule)

Besides baclofen, an antispastic drug, the others are all used for anaesthesia and pain management. Ziconotide is the only drug allowed exclusively for intrathecal administration. Attempts to administer rapamycin intrathecally are likewise known from publications: Geranton et al. Neurobiology of Disease, 2009 and Xu et al. The Journal of Neuroscience, 2011.

These two papers describe an intrathecal administration, not intraventricular, of rapamycin to mice, the aim being to understand the pathogenesis of neuropathic pain.

There is no known publication addressing the possibility of an intrathecal administration, much less an intraventricular one, of mTOR inhibitors, in particular everolimus, for the treatment of the pathologies to which the present invention relates.

Intrathecal administration of mTOR inhibitors, in particular everolimus, is carried out by means of a system which requires a simple and absolutely well-established surgical procedure. The administration system, preferably an intrathecal pump, can be maintained in place for several years, with minimum maintenance costs and care. The reliability of the device is fully satisfactory, as is its weight and size. The tank of the device can contain a sizeable amount of drug, so that the refill procedure can be carried out at reasonably long time intervals.

The drugs which are best tolerated for intrathecal administration are hydrophilic. Once in the cerebrospinal fluid (CSF), they are homogeneously diffused into the intrathecal space, penetrate into the parenchyma and reach the target cells. In part they are intercepted by the cerebral intraparenchymal venous system, and in part they return into the CSF. Then the CSF is captured in the subarachnoid space by Pacchioni's arachnoid granulations which protrude into the venous sinuses and deliver CSF into the bloodstream.

Therefore, the CSF and the solutes thereof are diluted in the new volume of distribution, following the usual catabolism and half-life. While in the CSF, the drug's concentration will depend on its volume of distribution, the speed of administration and CSF turnover; once in the bloodstream it is linked to the new volume of distribution, and for detoxification, the catabolic mechanisms of the filter organs (mTOR inhibitors are mainly degraded by the liver), and the half-life of rapamycin according to the FDA (http://www.fda.gov/ohrms/dockets/ac/99/slides/3529sle/sld002 .htm) is 62 hours.

On the other hand, lipophilic drugs (like almost all mTOR inhibitors) tend to diffuse into surrounding tissues and into the bloodstream (see, for example, Castro and Eisenah, Anesthesiology 1989). At the same time, a lipophilic drug can easily reach the target site in the central nervous system (CNS) in the pathologies concerned, above all if administered intraventricularly. In this case, a pulsatile or intermittent administration could enable the pharmacological effect to be obtained while limiting the increase in the plasma concentration.

Cerebrospinal fluid (CSF) is mainly produced by the choroid plexus in the ventricular system. It flows within it - in a pulsatile manner and in synchronism with the heartbeat - from the lateral ventricles to the fourth, then exits the ventricular system through the foramina of Luschka and Magendie and mainly flows into the subarachnoid space to reach Pacchioni's arachnoid granulations, which are situated in the upper part of the cranium and protrude into the venous sinuses; they intercept the CSF and transfer it into the venous blood of the sagittal sinus.

It also flows along the subarachnoid space around spinal cord and, very weakly, in the central spinal canal.

CSF can be considered an ultrafiltrate of plasma: in physiological conditions it contains ions, little protein and very few cells.

Its volume varies between 135 and 150 ml, but it is continuously produced at a speed of 500 ml/day. Therefore, it is completely replaced around 3.5 times a day.

If it is desired to maintain the concentration of a molecule in the cerebrospinal fluid (approx. 150 ml), hence in the central nervous system (approx. 1400 ml), it is necessary to administer it regularly into the cerebrospinal fluid. In order to maintain a therapeutic concentration of a drug in CSF, the amount necessary to reach the concentration in 500 ml of CSF and the volume of distribution thereof (CSF and CNS) must be regularly administered into the fluid over 24 hours.

Each day the same amount is gradually discharged into the bloodstream, where it is diffused in a larger volume of distribution. Therefore, the systemic concentration of the molecule will depend on its half-life and volume of distribution.

If the intrathecal drug is homogeneously distributed in about 1550 ml (CNS+CSF), in a therapeutic concentration, the same amount will gradually be transferred to the rest of the body and thus diluted 30-60 times, depending on the subject's body mass.

This daily dose is very different from the one necessary to reach any systemic pharmacological effect, so there is also a smaller likelihood of causing side effects.

Most mTOR inhibitors are not very water soluble. Their administration in an aqueous environment may require a microemulsion formulation (e.g. Bespinar et al, J Ocul Pharmacol Ther 2008) or encapsulation in particular PLGA nanoparticles (e.g. Tosi et al, J Neural Transm 2011).

The chemical characteristics of the chosen drug, in the formulations already described by other authors, can be such as to make it diffuse rapidly through a larger volume of distribution than desired. This diffusion could be slowed down by direct intraventricular and/or pulsatile administration. The aim of obtaining a local therapeutic effect (CNS) would in any case be achieved, while maintaining an absolutely tolerable systemic concentration.

### METHODS - GENERAL PART 1 SETUP OF DRUG ASSAY, CHOICE OF EXPERIMENTAL DOSAGE AND ANIMAL SURGERY

**Drug:** In order to demonstrate the effectiveness of the drug also in this new formulation and method of administration, we tested the effect of the mTOR inhibitor everolimus in mice (http://www.selleckchem.com/products/Everolimus(RAD001).html) , in view of its clinically demonstrated effectiveness and relatively brief half-life (28-32h, compared to more than double that time for rapamycin).

**Intraventricular infusion:** Under general anaesthesia obtained with Ketamine/Xylazine, Alzet osmotic pumps (http://www.alzet.com/products/guide_to_use/pump_selection.ht ml) were implanted subcutaneously on the back; a catheter leading from the pump reached the cranial vault, where it was connected to a connector (Brain Kit 3, Alzet) stereotaxically implanted in the cranium until reaching the lateral ventricle. These pumps are capable of infusing their content at a constant speed for 1-4 weeks.

### Assay of Everolimus via LC-MS

A quantitative analysis of everolimus in blood and tissue samples was performed with the isotopic dilution method using the LC-MS technique. The chromatographic system was the following: Agilent 1290 Infinity HPLC equipped with a binary pump and HiP Auto Sampler model G4226A. The injection volume was 2 µL, the analytical column used was a Phenomenex Kinetics PFP UHPLC column (Phenomenex, Milan, Italy) 5.0 × 2.1 mm, 1.7 µm, 100 Å, temperature controlled at 55 °C. A gradient elution was performed using as solvent A: water with 5mM ammonium formiate, solvent B: methanol with 0.1% formic acid. The elution profile was the following: solvent A 30%/ solvent B 70% for 3 minutes, solvent A 1%/ solvent B 99% from 3 to 6 minutes, at a flow velocity of 0.25 mL/min. This setup enabled an elution time of 2.5 minutes. Every injection was preceded by a cycle of washing the injector system with methanol.

Mass spectrometry was conducted using a Q-TOF MS detector model G6540A (Agilent, Germany) equipped with a Dual AJS ESI (electrospray ionization interface) system. The instrument parameters were the following: sheath gas flow: 7.5 mL/min, sheath gas temperature: 300 °C, gas flow: 6 L/min, gas temperature: 300 °C, nebulizer pressure: 35 psi, ion spray voltage: 5500 V.
Both everolimus and the isotopic internal standard were detected as ammonium adducts in the *positive ion* mode.

### Sample preparation and method validation

### Extraction from samples of whole blood

Everolimus was extracted from whole blood in 1 mL of a methanol solution containing 10% ammonium acetate in order to enable precipitation of most of the serum protein.

The internal standard used was isotopic 4d-2C¹³-everolimus added to the extraction solution at a concentration of 200 ng/mL.

The blood samples were freeze-dried and subsequently extracted. The extraction process was carried out in a sonication bath for approx. 10 minutes to ensure complete haemolysis and release of the drug from the cell material. The samples were centrifuged (10 minutes, 4000 rpm, 10 °C) and the supernatant was then separated from the pellet.

The solutions thus obtained were then purified by means of HybridSPE®-Phospholipid solid-phase extraction cartridges with a total volume of 1 ml (Sigma Aldrich, Milan) to eliminate the excess lipid material potentially present. The process was preliminarily validated both for everolimus and for the internal standard in solution and in matrices, providing a yield >98% w/v.

### Extraction from tissue samples of (brain)

The process of extraction from brain samples, divided into right and left hemispheres, was similar to the one for whole blood. The tissue samples were weighed and subsequently freeze-dried. After freeze-drying the samples were finely chopped to facilitate subsequent extraction. 1 mL of the methanol solution was then added to 1% v/v of ammonium formiate with 200 ng/mL of internal standard. The samples were then subjected to 10 minutes of sonication and vortexed to ensure complete extraction of the drug. Then followed centrifugation (10 minutes, 4000 rpm, 10 °C) and the supernatant was separated from the pellet. As previously described, the solutions were purified by means of HybridSPE®-Phospholipid solid-phase extraction cartridges with a total volume of 1 ml (Sigma Aldrich, Milan) to eliminate the excess lipid material. The samples were then immediately submitted to LC-MS analysis.

### Validation

Both the extraction method and the analytic method were validated in the following manner:
a) Extraction: the extraction from tissues and blood was validated by determining the yield for everolimus and for the internal standard under the same conditions both in solution and in the biological matrices. These yields were measured by contaminating samples from animals not treated with everolimus at a concentration of 100 ng/mL, while the internal standard was maintained at 200 ng/mL. The extraction and purification processes were then simulated and the amount was measured and compared to the theoretical amount.
b) Analysis: the analytical method was tested to determine LOD and LOQ and linearity in the response in the presence or absence of the biological matrices. A test calibration was performed in the range of 1-200 ng/mL everolimus to determine whether there was any significant interference of the matrix, maintaining the internal standard at the concentration of 200 ng/mL.

### Biochemistry - Western blot: Protein extraction and Western Blot analysis

The frozen mouse tissues were homogenized in T-PER solution (Pierce) containing a cocktail of protease inhibitors (Sigma-Aldrich) and a cocktail of phosphatase inhibitors (Sigma-Aldrich). The homogenized samples were sonicated 3 times for 15 sec (amplitude of 10 microns) at 4 °C using an MSE soniprep 150 sonicator (Sanyo), and subsequently centrifuged at 4 °C for 30 minutes at 16000g. The supernatant was recovered and the proteins quantified by means of a Bradford assay using BSA as the standard.

For the Western blot analysis, the proteins were separated by SDS-PAGE under reducing conditions according to the method of Laemmli. Subsequently, the proteins were transferred onto a PVDF membrane (Biorad), which was incubated with the following primary antibodies: anti-phospho-p70S6K (T³⁸⁹), anti-phospho-p70S6K (S³⁷¹), anti-p70S6K (Cell Signaling) and anti-actin (Sigma-Aldrich). After being washed with TBS-Tween (50 mM Tris, pH 7.5, NaCl 150 nM and 0.1% Tween 20), the membrane was incubated with one of the following secondary antibodies: anti-rabbit IgG conjugated to HRP (GE Healthcare) and anti-mouse IgG conjugated to HRP (GE Healthcare). The membrane was developed by means of ECL *prime* (GE Healthcare).

### mTOR activity

mTOR activity was measured using the K-LISA™ mTOR Activity kit (Calbiochem®), following the manufacturer's protocol. The kit is based on an ELISA activity assay which uses a fusion protein, GST-p70S6K, as a specific mTOR substrate. The mTOR substrate is first bound to a solid support and the mTOR-containing sample is incubated with ATP in the wells of a glutathione-coated 96-well plate. Active mTOR present in the sample phosphorylates the protein p70S6K bound to the plate at Thr³⁸⁹. The phosphorylated substrate is detected using a primary Anti-p70S6K-T³⁸⁹ antibody, followed by detection with a secondary antibody conjugated to HRP and addition of the TMB substrate. The associated activity is determined by reading absorbance at wavelengths of 450 and 595 nm.

### RESULTS PART 1 - GENERAL

### Search for appropriate dosage by mass spectrometry.

The brain concentration of everolimus obtained by intraparenchymal administration for one week at 3 different dosages was verified. They were chosen based on the highest doses published in animal trials (A = 2.5mg/Kg), the ones most commonly used in mice (B = 1.25mg/Kg), and the theoretically sufficient ones (C = 0,6125mg/Kg).

As shown in Table 1, we obtained different brain concentrations:
Brain conc 1: 11.5 mg/Kg
Brain conc 2: 25 mg/Kg
Brain conc 3: 41.7 mg/Kg

**Table 1- Blood and brain concentrations in 3 groups of mice treated intraperitoneally for a week with graduated doses of Ev.**

| **CD1 mice** | **Blood (ng/mL) (n=2)** | | | | | |
|---|---|---|---|---|---|---|
| | **0.6125 mg/kg** | | **1.25 mg/kg** | | **2.5 mg/kg** | |
| Males Females | 16±4 | | 42±4 | | 59±3 | |
| | 15±3 | | 25±5 | | 46±4 | |

| | **Brain (ng/0.25g)* (n=2)** | | | | | |
|---|---|---|---|---|---|---|
| | **0.6125 mg/kg** | | **1.25 mg/kg** | | **2.5 mg/kg** | |
| | Hemisphere dx | Hemisphere sx | Hemisphere dx | Hemisphere sx | Hemisphere dx | Hemisphere sx |
| Males Females | 14±3 | 9±2 | 37±5 | 25±8 | 52±6 | 44±9 |
| | 11±3 | 12±4 | 18±6 | 20±4 | 33±6 | 38±10 |

| | | | | | | |
|---|---|---|---|---|---|---|
| * the value is normalized to the weight of a hemisphere (0.25g) | | | | | | |

In any case the drug did not reach measurable levels in the blood. All dosages seemed to be very well tolerated based on an evaluation of body weight, spontaneous motility, posture and fur.

We then decided to look for the maximum tolerable dose by intraventricular administration of everolimus at concentrations 10 times higher than those shown in Table 1: A = 25mg/Kg, B = 12.5mg/Kg, C = 6.125mg/Kg.

After 3 days of treatment the mice treated with dose A demonstrated a reduction in spontaneous motility and a very slight weight reduction (none in posture or fur). Therefore, that dose was ruled out and the mice were sacrificed. The other 2 doses appeared to be well tolerated. For the subsequent assessment of therapeutic effectiveness in the different animal models, dose B (12.5 mg/Kg) was selected.

Table 2 shows the brain and blood concentrations found. Though they could not be quantified, the presence of metabolites deriving from the transformation of the drug has been noted in the table.

**Table 2: Blood and brain concentrations detected after one week of intracerebral administration at 3 different concentrations.**

| | **Sample** | **Conc / 250 mg brain** | **Brain field** | **Metabolites** | **Treatment duration Conc ng/g** |
|---|---|---|---|---|---|
| **A** | **Blood** | <1 | **T8 dx** | No metabolites | Treated 3 days 20,4ng/g |
| | | | **T8 sx** | Metabolite -42 | |
| | **Right hemisphere** | 5.3±2.8 | **T9 dx** | Metabolites -42, -26, +16 | |
| | | | **T9 sx** | No metabolites | |
| | **Left hemisphere** | 4.9±2.3 | **T10 dx** | Metabolite -42 | |
| | | | **T10 sx** | Metabolite -42 | |
| **B** | **Blood** | < 1 | **T4-dx** | Metabolites -42, -10 | Treated 14 days 12-16ng/g |
| | | | **T4- sx** | Metabolite -26 | |
| | **Right hemisphere** | 3.1±1.6 | **T5-dx** | No metabolites | |
| | | | **T5- sx** | Metabolites -42, -10 | |
| | **Left hemisphere** | 2.9±0.8 | **T6 dx** | Metabolite-42 | |
| | | | **T6 sx** | No metabolites | |
| **C** | **Blood** | <1 | **T2- dx** | No metabolites | Treated 14 days 11,8 ng/g |
| | | | **T2- sx** | No metabolites | |
| | **Right hemisphere** | 3.4±0.7 | **T1- dx** | No metabolites | |
| | | | **T1- sx** | No metabolites | |
| | **Left hemisphere** | 2.5±0.2 | **T3-dx** | Metabolite-42 | |
| | | | **T3- sx** | Metabolite-42 | |

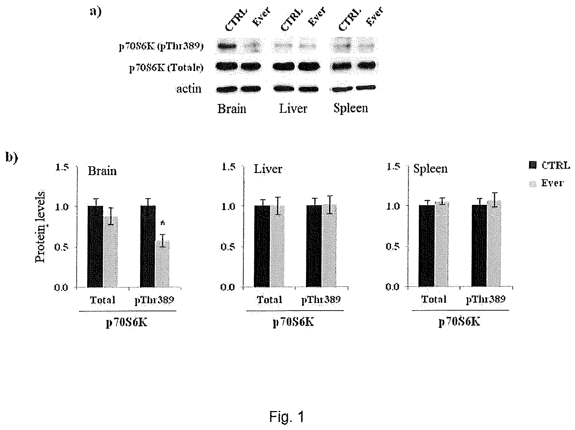

**Verification of mTOR inhibition.** We analyzed the actual inhibition of mTOR by WB. mTOR is a kinase with multiple substrates, one of which is p70-S6 kinase. Phosphorylation of the latter is unanimously considered a measure of mTOR activity. At the selected dose, the brain concentration reached did actually inhibit the enzymatic complex, as demonstrated by **figure 1****:** as could be expected, the p70-S6 kinase - normally very phosphorylated in the brain - is in a less phosphorylated form than in the controls. In parallel, p70-S6 kinase is normally phosphorylated in the spleen and liver.

The blood assays of everolimus with LC-MS had revealed a blood concentration in the therapeutic range in mice treated with intraperitoneal administration, whereas it was detectable only in traces (< 1mg/Kg) in the blood of mice that had received intrathecal treatment with everolimus. This corresponds to a local, but not systemic, inhibition of mTOR, as shown by WB, which determines the state of phosphorylation of p70-S6 kinase, proportional to the mTOR activity.

This renders any extracerebral pharmacological effect of the mTOR inhibitor absolutely improbable.

### PART 2 - SPECIAL: VERIFICATION OF EFFECTIVENESS IN MOUSE MODELS

We carried out trials in mouse models exemplifying 3 families of neurological diseases:
- **EAE** (Experimental Autoimmune Encephalomyelite, model of neuroinflammatory disease): SJL/j mice were immunized with two subcutaneous injections, performed 7 days apart, of proteolipid protein (PLP₁₃₉₋₁₅₁), Freund's adjuvant and inactivated Mycobacterium tuberculosis. The disease severity was rated with a clinical score: 0 = healthy; 1 = tail weakness; 2 = rear limb ataxia and/or paresis; 3 = rear limb paralysis and/or front limb paresis; 4 = tetraplegia; 5 = state of agony or death
- **AD** (Alzheimer's Disease, model of neurodegenerative disease): AD triple transgenic mouse (3xTg-AD) supplied by Jackson Laboratories (http://jaxmice.jax.org/strain/004807.html) Their memory was evaluated using the Passive Avoidance and Radial Maze tests;
- **TSC** (Tuberous sclerosis complex, model of neuro-oncologic disease): TSC1 mouse with TSC1 gene within a flox cassette, supplied by Jackson Laboratories (http://jaxmice.jax.org/strain/005680.html)

### EAE

30 SJL/j mice were immunized and started developing the disease after 11 days. As they developed the neurological deficit and reached a score of 1, they were operated on and an Alzet 1004 pump containing everolimus or a vehicle was positioned. The purpose of this was to represent the usual clinical situation in which the patient goes to the doctor's on the occasion of the first attack. Generally speaking, a diagnosis is made and a high-dose steroid therapy is applied, with good regression of the symptoms, but in the majority of cases other attacks will occur and they will be followed by an increasingly less satisfactory regression, with a gradual increase in residual disability.

In our experimental set, the weight and clinical score of affected mice were monitored daily.

We were able to observe a reduction in the severity of the first attack, which was however followed by another 2 attacks of a severity that was not much milder than in the controls. However, while the controls continued to manifest recurring attacks with increasingly greater residual disability, after the third attack the treated mice no longer manifested attacks and fully recovered their motor abilities (score 0) and body weight.

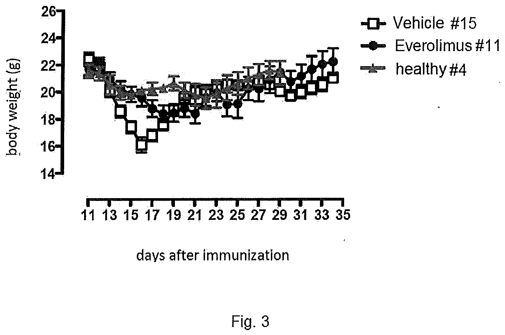

**Figure 3** shows the trend in body weight of the SJL mice. Initially, weight decreased as the disease progressed, but then tended to recover after the first, more violent attacks. Moreover, the implant operation in itself provokes a reduction in weight for several days.

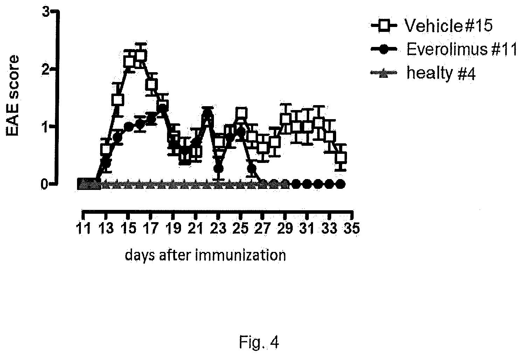

**Figure 4** shows the trend in the clinical score. After the 3rd attack, the drug-treated mice did not manifest any others, whereas the vehicle-treated mice continued to have them. At the end of the 4th week the mice were sacrificed and biochemical and immunopathological analyses were performed.

This demonstrates that intrathecal administration of Everolimus has a powerful local immunosuppressive effect. Though it is true that the method involves a certain degree of invasiveness and is absolutely not an etiologic remedy, the clinical effectiveness is wholly comparable to that of systemic administration, without systemic immunosuppressive effects, and this makes the method a therapeutic option of primary clinical interest.

### AD (Alzheimer's Disease)

The mouse model selected is considered one of those which best represent the human condition. Starting from 6 months of age, in fact, it manifests a progressive accumulation of intra- and extracellular amyloid-beta, neurofibrillary tangles and neuronal loss, as in man. These neuropathological characteristics are associated with progressive cognitive impairment, observable above all with a parallel impairment of mnestic performance.

Memory impairment, likewise a key symptom of AD, is measured using various tests. Among them, the universally accepted ones, which we ourselves used, are the Passive Avoidance, Radial Maze and Water Maze tests.

Passive Avoidance: the passive avoidance apparatus consists of two compartments separated by a sliding door. The smaller compartment is lit, the larger one is kept dark and the electric shocks could be delivered from the floor (2 × 0.4 mA, 50 Hz, 2 s in a 5 s interval). On the training day, each mouse was placed in the lit compartment, far from the dark compartment. The sliding door was opened when the mouse turned around and foot shocks were delivered when the mouse stepped into the dark compartment with all four feet. Memorization was tested 24 hours later and the latency to cross was recorded (time limit of 180 s).

Radial Maze: the mice were reduced in weight and the body weight was maintained at 85% of the normal weight by feeding them pre-measured amounts of food. The maze had 8 identical 37-centimer-long arms branching out from a 7 × 8 cm platform. The animals were subjected to one test per day and their training ended either when they learned the right choices (up to a maximum of 15 choices) or 15 min. had elapsed. A choice was defined as the positioning of all 4 feet on an arm of the maze.

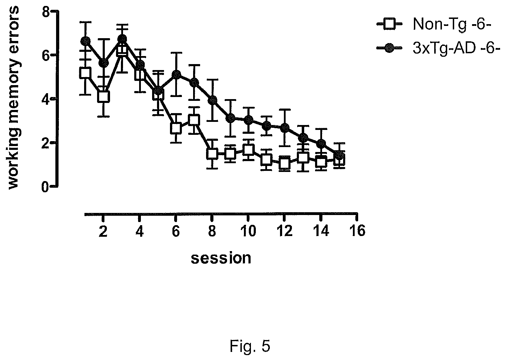

Figure 5 shows the performance of AD mice versus healthy controls at 6 months when subjected to the Radial Maze test. In a training process that involves daily sessions, this test measures the number of errors in recognizing a fixed path.

Whereas healthy mice showed only a few occasional errors after 8 days, this result was reached by the AD model after 15 days.

We applied intraventricular catheters and everolimus-secreting osmotic pumps implanted subcutaneously on the back of 12 AD mice for 4 weeks. We infused a vehicle to an equal number of mice. We injected everolimus into 10 healthy mice and, finally, the vehicle into another 10. The appropriate pump was replaced after 4 weeks and kept in place for another 4. Then mnestic performances were tested.

Whereas in the EAE mice the local application of Ev showed an effective local immunosuppressive activity, in this case, with the same dosage, a relatively novel pharmacological activity of the mTOR inhibitor was observed, one that was not taken into consideration until a few years ago: an increase in autophagy.

This was thoroughly analyzed by the group of S. Oddo (e.g. Caccamo JBC 2010). We would like to point out here that even should AD have a systemic involvement, this has certainly never been demonstrated to have any clinical relevance. A therapy with an mTOR inhibitor would however have side effects secondary to the immunosuppressive effect (see, for example, Krueger DA, NEJM 2010), which an elderly subject would find hard to tolerate.

Though invasive and not etiologic, the method proposed here shows such effectiveness versus such limited risks that we feel the cost/benefit ratio weighs decidedly in favour of the denominator.

### TS (Tuberous Sclerosis)

There are no available animal models which reproduce the main neurological characteristic of human TSC, subependymal giant cell astrocytomas (SEGA). These represent the leading cause of death in patients, and reducing their volume through oral administration of an mTOR inhibitor is the first endpoint of clinical trials on this subject.

We know, however, that the side effects of this route of administration are very severe.

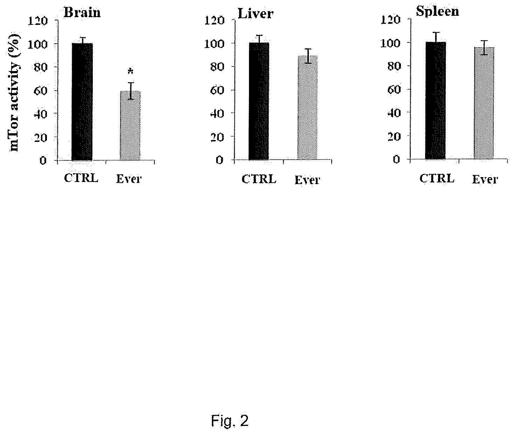

From the evidence presented in the General Part we know we are capable of reaching locally, within the cranial theca, a concentration of inhibitor greater than that reached in the same region through systemic administration (Table 2). From there we also know that the inhibitor present here is capable of inhibiting the mTOR signalling pathway (**Figures 1** **and** **2**). We have seen that this concentration is capable of performing an immunosuppressive action locally (**Figure 4**), while remaining in the blood at unassayable levels (table 2), and without affecting the normal activity of mTOR in peripheral organs (Figures 1 and 2).

## Claims

1. An mTOR inhibitor for use in the treatment, by intrathecal administration, of pathologies selected from: tuberous sclerosis, Alzheimer's disease, primary progressive aphasia and multiple sclerosis, wherein the mTOR inhibitor is everolimus.

2. The inhibitor for use according to claim 1, wherein said intrathecal administration is an intraventricular administration within the lateral ventricles.

3. The inhibitor for use according to claim 1 or 2, wherein said intrathecal administration is a pulsed administration, preferably at regular intervals, or else a continuous administration.

4. The inhibitor for use according to any one of claims 1 to 3, wherein said inhibitor is formulated in microemulsions.

5. The inhibitor for use according to any one of claims 1 to 3, wherein said inhibitor is encapsulated in nanoparticles of poly(lactic-co-glycolic acid) (PLGA), preferably modified in such a way as to be able to cross the blood-brain barrier.

6. The inhibitor for use according to any one of claims 1 to 5, wherein a mechanical or electromechanical device, preferably an electromechanical infusion pump, is used for said intrathecal administration.

7. The inhibitor for use according to claim 6, wherein said mechanical or electromechanical device is refillable.

## Patentansprüche

1. mtor-Inhibitor zur Verwendung bei der Behandlung, durch intrathekale Administration, von Pathologien ausgewählt aus: tuberöser Sklerose, Alzheimer-Krankheit, primär progredienter Aphasie und multipler Sklerose, wobei der mTOR-Inhibitor Everolimus ist.

2. Inhibitor zur Verwendung nach Anspruch 1, wobei die intrathekale Administration eine intraventrikuläre Administration innerhalb der lateralen Ventrikel ist.

3. Inhibitor zur Verwendung nach Anspruch 1 oder 2, wobei die intrathekale Administration eine gepulste Administration, vorzugsweise in regelmäßigen Intervallen, oder auch eine kontinuierliche Administration ist.

4. Inhibitor zur Verwendung nach einem der Ansprüche 1 bis 3, wobei der Inhibitor in Mikroemulsionen formuliert wird.

5. Inhibitor zur Verwendung nach einem der Ansprüche 1 bis 3, wobei der Inhibitor in Nanopartikeln von Poly(lactid-co-glycolid) (PLGA) eingekapselt ist, vorzugsweise derart modifiziert, dass er in der Lage ist, die Blut- Gehirnbarriere zu durchdringen.

6. Inhibitor zur Verwendung nach einem der Ansprüche 1 bis 5, wobei eine mechanische oder elektromechanische Vorrichtung, vorzugsweise eine elektromechanische Infusionspumpe, für die intrathekale Administration verwendet wird.

7. Inhibitor zur Verwendung nach Anspruch 6, wobei die mechanische oder elektromechanische Vorrichtung nachfüllbar ist.

## Revendications

1. Inhibiteur de mTOR utilisé dans le traitement, par administration intrathécale, de pathologies sélectionnées parmi : la sclérose tubéreuse de Bourneville, la maladie d'Alzheimer, les aphasies progressives primaires et la sclérose en plaques, dans lequel l'inhibiteur de mTOR est un évérolimus.

2. Inhibiteur utilisé selon la revendication 1, dans lequel ladite administration intrathécale est une administration intraventriculaire à l'intérieur des ventricules latéraux.

3. Inhibiteur utilisé selon la revendication 1 ou 2, dans lequel ladite administration intrathécale est une administration pulsée, de préférence à intervalles réguliers, ou sinon une administration continue.

4. Inhibiteur utilisé selon l'une quelconque des revendications de 1 à 3, dans lequel ledit inhibiteur est élaboré sous forme de microémulsions.

5. Inhibiteur utilisé selon l'une quelconque des revendications de 1 à 3, dans lequel ledit inhibiteur est encapsulé dans des nanoparticules d'acide poly (lactique-co-glycolique) (PLGA), de préférence modifié de manière à pouvoir traverser la barrière hématoencéphalique.

6. Inhibiteur utilisé selon l'une quelconque des revendications de 1 à 5, dans lequel un dispositif mécanique ou électromécanique, de préférence une pompe à perfusion, est utilisé pour ladite administration intrathécale.

7. Inhibiteur utilisé selon la revendication 6, dans lequel ledit dispositif mécanique ou électromécanique est rechargeable.
